# EUROPEAN PATENT SPECIFICATION

(11) **EP 2 010 914 B1**
(45) Date of publication and mention of the grant of the patent: **15.10.2014**
(21) Application number: 07732396.2
(22) Date of filing: 12.04.2007
(51) Int. Cl.: G01N 33/569, G01N 33/553, G01N 33/543

(54) **METHOD FOR ASSAYING ACTIVE ANTHRAX PROTECTIVE ANTIGENS**
VERFAHREN ZUR BESTIMMUNG VON AKTIVEN ANTIGENEN DIE VOR MILZBRAND SCHÜTZEN
MÉTHODE DE DOSAGE D'ANTIGENES L'ANTHRAX ACTIVES

(30) Priority: 13.04.2006 GB 0607462
(43) Date of publication of application: 07.01.2009
(73) Proprietor: PharmAthene UK Limited, Billingham Cleveland TS23 1XN (GB)
(72) Inventor: WATKINSON, Allan, Guisborough TS14 6GG (GB); WILSON, Robert, Billingham TS23 1XN (GB); DOUGLASS, Steven, Birmingham TS23 1YN (GB); MYERS, James, Philip, Birmingham TS23 1YN (GB)
(74) Representative: Williams, Gareth Owen
(86) International application number: PCT/GB2007/001353
(87) International publication number: WO 2007/122373

(56) References cited:
- WO-A-2004/073735
- GB-A- 1 561 042
- KATZ J B ET AL: "IN VITRO ASSESSMENT OF VIRAL ANTIGEN CONTENT IN INACTIVATED ALUMINUM HYDROXIDE ADJUVANTED VACCINES" JOURNAL OF VIROLOGICAL METHODS, AMSTERDAM, NL, vol. 25, no. 1, 1989, pages 101-108, XP001009902 ISSN: 0166-0934
- KATZ J B ET AL: "Radioimmunoassay of adjuvant-associated porcine parvovirus using a monoclonal antibody in a nitrocellulose membrane system", JOURNAL OF VIROLOGICAL METHODS, ELSEVIER BV, NL, vol. 12, no. 3-4, 1 December 1985 (1985-12-01), pages 193-198, XP023697310, ISSN: 0166-0934, DOI: 10.1016/0166-0934(85)90129-6 [retrieved on 1985-12-01]
- D. ZHU ET AL: "Development of a Direct Alhydrogel Formulation Immunoassay (DAFIA)", JOURNAL OF IMMUNOLOGICAL METHODS, ELSEVIER SCIENCE PUBLISHERS B.V.,AMSTERDAM, NL, vol. 344, no. 1, 15 May 2009 (2009-05-15), pages 73-78, XP027566139, ISSN: 0022-1759 [retrieved on 2009-03-27]

## Description

The present invention concerns a method for assaying anthrax protective antigens and more particularly to a method for measuring the viability or potency of said antigen.

Many vaccine drug products comprise an antigen adsorbed onto a solid inorganic support, especially a colloidal aluminium hydroxide, such as Alhydrogel (RTM). The adsorption onto the support means the antigen is not amenable to many conventional assay methods, such as immunoassays. Immunoassay methods have been proposed for assaying such drug products, but these rely upon either the antigen desorbing from the support, thereby risking damaging the antigen, or indirect methods of analysis, wherein a known amount of antibody is contacted with the antigen, the residual antibody determined, and hence the quantity of antigen determined by difference. For many antigens, and particularly in the case of anthrax protective antigen, the strength of the adsorption increases with storage, rendering the risk of damage on desorption even greater. It would therefore be desirable to identify alternative methods for assaying antigens adsorbed onto solid supports, especially a method which can serve as a reliable measure of viability or potency, and can be applied in vitro.

Viral antigen enzyme immunoassay (ELISA) is described in Kostenbader and Cliver, 1983, J Virol Methods 7, 253 to 257; and Furuya et al, 1984, J Virol methods 9, 193 to 191; with a requirement to disassociate antigen from adjuvant being described in Doel and Staple, 1982, J Biol Stand 10, 185 to 195; Barteling et al, 1983, J Biol Stand 11, 297 to 304; White and Hem, 1984, J Parenter Sci Technol, 38, 2 to 10; and Katz, 1987, Vet Res Com 11, 83 to 92.

GB1561042 describes use of microspheres for detection of the presence of microorganisms in biological samples. WO2004/073735 describes various anthrax vaccines compositions. Katz et al, J Virol Methods, 25, 101 to 108 describes immunological assays for adjuvanted inactivated vaccines.

According to the present invention there is provided a method for assaying the quantity of active anthrax protective antigen present on a pharmacologically-acceptable colloidal adjuvant, comprising: a) contacting a suspension of a colloidal adjuvant-adsorbed active protective antigen with a solution or suspension of a detectably-labeled primary antibody to the active anthrax protective antigen to form an active anthrax protective antigen-antibody complex and measuring the detectable label relative to that of a sample of standard active drug product; or b) contacting a suspension of a colloidal active anthrax protective adjuvant-adsorbed antigen with a solution or suspension of a primary antibody to the active anthrax protective antigen to form an active protective antigen-antibody complex and contacting said antigen-antibody complex with a detectably-labeled probe for the active protective antigen-antibody complex and measuring the detectable label on the probe relative to that of a sample of standard active drug product, thereby determining the quantity of active anthrax protective antigen.

Although not in accordance with the present invention as claimed, anthrax Antigens which can be assayed by the method of the present invention include components of subunit vaccines, usually recombinant protein-based subunit vaccines. Examples of such antigens include Hepatitis B protective antigens, Herpes Simplex Virus antigens, Influenza antigens, Congenital cytomegalovirus (CMV) antigens, Tuberculosis antigens, HIV antigens, Diphtheria antigens, Tetanus antigens, Pertussis antigens and Yersinia pestis protective antigens, such as antigens comprising one, two or more antigenic proteins. Most preferably, the antigen is an anthrax protective antigen.

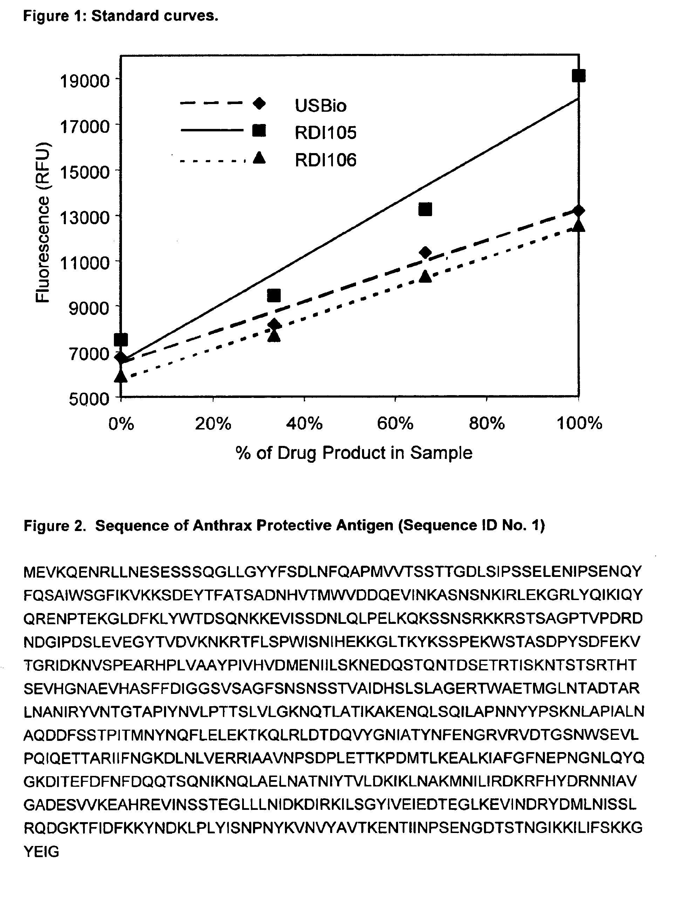

Anthrax protective antigens which can be assayed by the method according to the present invention are well known in the art. Preferably, the anthrax protective antigen is recombinant protective antigen as described in WO02/04646, such as the antigen having the sequence given in Figure 2 (Sequence ID No. 1).

Examples of solid supports onto which the antigen is adsorbed are preferably pharmacologically-acceptable adjuvants, especially colloidal adjuvants, such as calcium phosphate, aluminium phosphate, such as the colloidal aluminium phosphate available under the trade name "AdjuPhos (RTM)" and most preferably aluminium oxyhydroxide, most commonly known by the name "Alhydrogel (RTM)". Aluminium oxyhydroxide is also known as aluminium hydroxide or alum.

The antigen adsorbed on the solid support is assayed as a suspension in a liquid, most often an aqueous liquid. Most commonly the liquid is an aqueous buffer, such as a buffered saline solution, especially a phosphate-buffered saline solution or a Tris-buffered saline solution. When a buffer is employed, it preferably has a pH in the range of from 7 to 8, most preferably around 7.5. When the antigen adsorbed on the solid support is not already in the form of a suspension, such suspensions can readily be prepared using method known in the art.

Antibodies which can be employed as either primary antibodies or as probes in the method of the present invention are preferably monoclonal antibodies. In many embodiments, the primary antibody is selected to be an antibody for a region of anthrax protective antigen which is indicative of the activity of the antigen, and preferably is an antibody for Domain 4 of anthrax protective antigen. Especially preferred antibodies are antibodies which bind to the regions defined by amino acids 636 to 653 inclusive (IRKILSGYIVEIEDTEGL, Sequence ID No. 2), 660 to 677 inclusive (RYDMLNISSLRQDGKTFI, Sequence ID No. 3) or 676 to 690 inclusive (FIDFKKYNDKLPLYI, Sequence ID No. 4) of the antigen having the Sequence ID No. 1. Most preferred antibodies are antibodies for regions which comprise the aspartic acid residue at position 684 of the antigen having the Sequence ID No. 1. Methods of identifying suitable antibodies are known in the art. Examples of suitable primary antibodies include anti-protective antigen monoclonal antibody clone 2B18 (mouse) commercially available from United States Biological, anti-protective antigen monoclonal antibody clone RDI-TRK3BA16-C3 (clone C3; mouse) commercially available from RDI-Fitzgerald, anti-protective antigen monoclonal antibody clone RDI-TRK3BA16-105 mouse mAb commercially available from RDI-Fitzgerald, and anti-protective antigen monoclonal antibody clone RDI-TRK3BA16-106 mouse mAb commercially available from RDI-Fitzgerald.

The primary antibody may be employed as a suspension. Preferably, the primary antibody is employed as a solution, most often an aqueous solution, and most commonly as a solution in an aqueous buffer, such as a buffered saline solution, for example a phosphate-buffered saline solution or a Tris-buffered saline soon. In certain embodiments, the buffer employed may comprise a blocking buffer. When an aqueous buffer is employed, it preferably has a pH in the range of from 7 to 8, most preferably around 7.5. In many embodiments, the primary antibody is employed in a molar excess to the anticipated amount of antigen, such as up to a 2100 times excess, commonly up to a 500 times excess.

Probes for the primary antibody/antigen complex that may be employed include secondary antibodies for the primary antibody/antigen complex, and immunoglobulin binding proteins such as Protein A and Protein G. Preferably, secondary antibodies for the primary antibody/antigen complex are employed.

When a secondary antibody for the primary antibody/antigen complex is employed, the secondary antibody is preferably an antibody for the primary antibody portion of the complex. In this case, selection of the secondary antibody will depend upon the nature of the primary antibody, and in particular where the primary antibody was produced. Examples of combinations of secondary antibodies with primary antibodies are well known in the art. In many cases, when the primary antibody is monoclonal, it is often produced in mice, and when polyclonal, in goats or rabbits. Preferred secondary antibodies include IgG anti-mouse antibody and IgG anti-rabbit antibody.

The secondary antibody may be employed as a suspension. Preferably, the secondary antibody is employed as a solution, most often an aqueous solution, and most commonly as a solution in an aqueous buffer, such as a buffered saline solution, especially a phosphate-buffered saline solution or a Tris-buffered saline solution. In certain embodiments, the buffer employed may comprise a blocking buffer. When an aqueous buffer is employed, it preferably has a pH in the range of from 7 to 8, most preferably around 7.5. In many embodiments, the secondary antibody is employed in a molar excess to the anticipated amount of primary antibody/antigen complex, such as up to a 100 times excess, commonly up to a 20 times excess, for example in the range of from a 1.5 to 10 times excess.

Detectable labels, and the methods for detecting them are well known in the art, and include radiolabels, such as ¹²⁵I labels; fluorescent labels, such as fluorescein and FITC labels; biotinylation with avidin or streptavidin detection; and enzymic labels, such as horseradish peroxidase and alkaline phosphatase labels. The labels may be attached by methods known in the art.

The contact between the solid-supported antigen and the antibody/antibodies may conveniently be accomplished in a centrifuge tube. The use of a centrifuge tube allows convenient separation of the solid-supported antigen and solid-supported antibody/antigen complex from solutions or suspensions by centrifugation. In other embodiments, the contact between the solid-supported antigen and the antibody/antibodies may conveniently be accomplished in a well plate, commonly a 96-well plate. The dimensions of the well plate are conveniently selected so that the separation of the solid-supported antibody and solid-supported antibody/antigen complex from solutions or suspensions can be achieved by spinning down the plate such that the solid-supported antigen or antibody/antigen complex remains in the wells whilst the solutions or suspensions are removed, for example by decanting or by pipetting. Other methods of contact known in the art may also be employed if desired, for example by using filter plates (well plates in which the base of the well is a filter membrane) where separation can be achieved by filtration. In certain embodiments, good results have been achieved by removing fixed volumes of supernatants, especially by pipette, and most preferably by multi-channel pipette.

The method according to the present invention commonly employs washing solutions and blocking agents in accordance with conventional practice for immunoassays.

Blocking agents which can be employed are well known in the art. Preferred blocking agents include non-ionic surfactants, such as polyalkoxylated sorbitan alkanoates, for example Tween 20™, and non-specific proteins such as powdered milk protein (casein) and bovine serum albumin (BSA). A preferred blocking agent comprises a mixture of polyalkoxylated sorbitan alkanoate and casein and optionally BSA. In many embodiments, good results have been achieved using the blocking buffer solution commercially available from Invitrogen as part of its Western Breeze™ kit.

The present invention is illustrated without limitation by the following examples.

### Example 1

### Reagents

Sample - Drug product having an anthrax protective antigen concentration of 0.2mg/mL was prepared as follows. A solution of 0.4mg/mL anthrax protective antigen was prepared by diluting 0.5mL of anthrax protective antigen solution in phosphate-buffered saline (PBS, pH 7.4) with a further 1.125mL of PBS. 0.26mL of alhydrogel (RTM) suspension (Sigma A-8222) was mixed with 0.74mL of 1.22% saline. To this was added 1mL of the 0.4mg/mL anthrax protective antigen solution followed by gentle mixing for 1hour at room temperature to give the drug product.

Primary antibody - as detailed in Table 1 below.

Secondary antibody - Sigma F0257 anti-mouse IgG (whole molecule)-FITC antibody produced in goat (20µg/mL i.e. 100µL of conc. antibody in 10mL of antibody diluent).

Blocking, antibody wash and antibody diluent solutions - from the Invitrogen Western Breeze kit and are prepared as per the Western Breeze kit instructions for PVDF membranes.

A Falcon 96-well tissue culture treated plate (transparent polystyrene) with a well volume of approximately 350µL was used. 250µL of blocking solution plus 5µL of thoroughly mixed samples were loaded into wells in a randomised order. A normal single channel pipette of the Gilson type was used for the addition of samples to the plate. Additions of other solutions were done using a multi-channel pipette.

Mixing was done on a microplate shaker platform at a shaking speed of 500rpm to avoid splashing. Spinning down was done using a Centurion centrifuge with a microplate rotor.

Supernatants were removed by removing the lid, turning the plate upside down to an angle of ca. 20° from the horizontal and gently shaking it up and down at a rate of approximately 1 cycle per second. The plate must not be vigorously shaken or flicked as the solid is only loosely bound to the base. Excessively vigorous shaking of the plate can dislodge some solid, reducing assay accuracy.

The sequence employed was as follows:

### Immunoassay Procedure:

(1) Add 250µL blocking solution to each well
(2) Add 5µL of sample to each well
(3) Mix on the shaker platform at 500rpm for 30 mins
(4) Spin down at 3krpm for 5mins
(5) Remove supernatant
(6) Add 200µL of Primary Antibody Solution
(7) Mix on the shaker platform at 500rpm for 1 hour
(8) Spin down at 3krpm for 5mins
(9) Remove supernatant
(10) Add 250µL of Antibody Wash
(11) Spin down at 3krpm for 5mins
(12) Remove supernatant
(13) Add 250µL of Antibody Wash
(14) Mix on the shaker platform at 500rpm for 30 mins
(15) Spin down at 3krpm for 5mins
(16) Remove supernatant
(17) Add 250µL of Water
(18) Spin down at 3krpm for 5mins
(19) Remove supernatant
(20) Add 200µL of Secondary Antibody Solution
(21) Mix on the shaker platform at 500rpm for 1 hour
(22) Spin down at 3krpm for 5mins
(23) Remove supernatant
(24) Add 250µL of Antibody Wash
(25) Spin down at 3krpm for 5mins
(26) Remove supernatant
(27) Add 250µL of Antibody Wash
(28) Mix on the shaker platform at 500rpm for 30 mins
(29) Spin down at 3krpm for 5mins
(30) Remove supernatant
(31) Add 200µL of 8M Urea to solubilise fluorescent label
(32) Mix on the shaker platform at 500rpm for 30 mins
(33) Read the fluorescence of the wells using a plate reader.

The fluorescence was read using a Bio-TEK Synergy HT with an excitation wavelength of 485nm and an emission wavelength of 528nm.

To illustrate the utility of the method of the present invention as a measure of viability, the immunoassay procedure was used to assay three samples of anthrax protective antigen drug product. Two samples were products of storage stability trials and were known to have lost activity, and one sample was a sample from a storage stability trial known to have retained acceptable activity. As standards, a dilution series prepared from a recent batch of anthrax protective antigen drug product was prepared, together with a blank comprising the drug product diluent but no anthrax protective antigen. The three assay samples and the standard were all of the same formulation.

Three different primary antibodies were employed, as detailed in Table 2, each having been shown to bind to anthrax protective antigen domain 4.

Table 3 shows the samples which were assayed.

Six wells of each sample were assayed, two cells each of the three primary antibodies.

A Bio-TEK Synergy HT Plate reader was used for the fluorescence measurements.

**Table 1. Primary Antibodies employed**

| **Abbreviation** | **Antibody** | **Concentration used** |
|---|---|---|
| USBio | United States Biological B0003-05J Clone 2B18 mouse mAb | 10µg/mL i.e. 50µL of conc. antibody in 10mL of antibody diluent |
| RDI105 | RDI-TRK3BA16-105 mouse mAb | 10µg/mL i.e. 12µL of conc. antibody in 10mL of antibody diluent |
| RDI106 | RDI-TRK3BA16-106 mouse mAb | 10µg/mL i.e. 24.4µL of conc. antibody in 10mL of antibody diluent |

**Table 2. Samples**

| **Sample Name** | **Description** |
|---|---|
| 100% Std | Standard active drug product |
| 66% Std | Standard active drug product diluted to 66% activity with BPS formulated diluent |
| 33% Std | Standard active drug product diluted to 33% activity with BPS formulated diluent |
| Blank | BPS formulated diluent without any anthrax protective antigen |
| Sample 1 | Drug product from storage known to have retained acceptable activity |
| Sample 2 | Sample from a stability study know to have lost potency |
| Sample 3 | Sample from a second stability study know to have lost potency |

### Results

The average fluorescence results for the three standard solutions and blank (shown in Table 3 below) were plotted to give standard curves for each antibody. The standard curves are shown in Figure 1 below.

**Table 3. Fluorescence Results for Standard solutions and Blank**

| Primary Antibody→ | **USBio** | **RDI105** | **RDI 106** |
|---|---|---|---|
| **Sample ↓** | **FI(RFU)** | **FI(RFU)** | **FI(RFU)** |
| **100%Std (Batch 804151)** | 13161 | 19087 | 12506 |
| **66% Std** | 11333 | 13234 | 10293 |
| **33% Std** | 8175 | 9459 | 7704 |
| **Blank (i.e. diluent)** | 6737 | 7520 | 5910 |

Using the mean fluorescence figures obtained for each of samples 1 to 3, the relative activity of each of the samples could be determined. The mean fluorescence figures, and percentage activity are given in Table 4 below.

**Table 4. Fluorescence Results and Activity for Samples 1 to 3**

| Primary Antibody→ | **USBio** | | **RDI 105** | | **RDI 106** | |
|---|---|---|---|---|---|---|
| **Sample** ↓ | **FI(RFU)** | **% Activty** | **FI(RFU)** | **% Activty** | **FI(RFU)** | **% Activty** |
| **1** | 12121 | 84% | 15669 | 79% | 11032 | 79% |
| **2** | 5700 | -12% | 7582 | 9% | 5756 | 0% |
| **3** | 4033 | -36% | 4011 | -22% | 3520 | -33% |

From the results, it can be seen that sample 1, which was known to have acceptable activity, gave a very good response compared with the active standard, whereas the samples known to have lost potency gave extremely poor results. This demonstrates that the method according to the present invention can be employed as an in vitro viability/potency assay for anthrax protective antigen.

### Example 2

Samples of drug product from a 40°C storage stability trials and a control stored at 2-8°C were analysed by a variation on the method given in Example 1 above. The standard curve was generated by using a dilution series from standard material of known viability prepared by the same method which had been stored at 2-8°C for 7 days.

All drug product and alhydrogel suspensions were handled by positive displacement pipettes.

The primary antibody employed was RDI-TRK3BA16-C3 (clone C3; mouse - commercially available from RDI-Fitzgerald) at 0.01 mg/ml concentration. The secondary antibody employed was Sigma F0257 anti-mouse IgG (whole molecule)-FITC antibody produced in goat at 6.246mg/ml concentration (15µl of supplied concentrate per 1ml of diluent).

Blocking buffers and antibody washes employed were obtained from a Western breeze kit, supplied by Invitrogen.

The samples were diluted 1:2 with 0.26% alhydrogel (RTM) in PBS, and this solution further diluted 1:24 with blocking buffer 125µl of solution was added to wells of a Falcon 96-well tissue culture treated plate (transparent polystyrene) with a well volume of approximately 350L.

The assay then followed the following procedure:
(1) Top up wells with 125µl blocking buffer.
(2) Mix on the shaker platform at 500rpm for 30 mins
(3) Spin down at 3krpm for 5mins
(4) Remove 150µl supernatant using a multi-channel pipette
(5) Add 200µL of Primary Antibody Solution
(6) Mix on the shaker platform at 500rpm for 1 hour
(7) Spin down at 3krpm for 5mins
(8) Remove 200µl supernatant using a multi-channel pipette
(9) Add 200µL of Antibody Wash
(10) Spin down at 3krpm for 5mins
(11) Remove 200µl supernatant using a multi-channel pipette
(12) Add 200µL of Antibody Wash
(13) Mix on the shaker platform at 500rpm for 5 mins
(14) Spin down at 3krpm for 5mins
(15) Remove 200µl supernatant using a multi-channel pipette
(16) Add 200µL of blocking buffer
(17) Spin down at 3krpm for 5mins
(18) Remove 200µl supernatant using a multi-channel pipette
(19) Add 200µL of blocking buffer
(20) Spin down at 3krpm for 5mins
(21) Remove 200µl supernatant using a multi-channel pipette
(22) Add 200µL of Secondary Antibody Solution
(23) Mix on the shaker platform at 500rpm for 1 hour
(24) Spin down at 3krpm for 5mins
(25) Remove 200µl supernatant using a multi-channel pipette
(26) Add 200µL of Antibody Wash
(27) Spin down at 3krpm for 5mins
(28) Remove 200µl supernatant using a multi-channel pipette
(29) Add 200µL of Antibody Wash
(30) Mix on the shaker platform at 500rpm for 5 mins
(31) Spin down at 3krpm for 5mins
(32) Remove 200µl supernatant using a multi-channel pipette
(33) Add 200µL of blocking buffer
(34) Spin down at 3krpm for 5mins
(35) Remove 200µl supernatant using a multi-channel pipette
(36) Add 200µL of blocking buffer
(37) Spin down at 3krpm for 5mins
(38) Remove 200µl supernatant using a multi-channel pipette

The fluorescence was read using a Bio-TEK Synergy HT with an excitation wavelength of 485nm and an emission wavelength of 528nm.

The results of the assay showed that the control sample stored at 2-8°C had an activity equivalent to 458.8µl antigenic rPA/ml of drug product, whereas the sample stored at 40°C had zero antigenic rPA remaining.

## Claims

1. A method for assaying the quantity of active anthrax protective antigen present on a pharmacologically-acceptable colloidal adjuvant, comprising:
a) contacting a suspension of a colloidal adjuvant-adsorbed active anthrax protective antigen with a solution or suspension of a detectably-labeled primary antibody to the active anthrax protective antigen to form an active anthrax protective antigen-antibody complex and measuring the detectable label relative to a sample of standard active drug product; or
b) contacting a suspension of a colloidal active anthrax protective adjuvant-adsorbed antigen with a solution or suspension of a primary antibody to the active protective antigen to form an active anthrax protective antigen-antibody complex and contacting said active anthrax protective antigen-antibody complex with a detectably-labeled probe for the active anthrax protective antigen-antibody complex and measuring the detectable label on the probe relative to a sample of standard active drug product, thereby determining the quantity of active anthrax protective antigen.

2. The method according to claim 1, wherein the antigen is a component of a sub-unit vaccine.

3. The method of claim 1 3, wherein the antigen comprises the amino acid sequence of SEQ ID NO: 1.

4. The method according to claim 1 3, wherein the primary antibody is an antibody for Domain 4 of anthrax protective antigen.

5. The method according to claim 1, wherein said colloidal adjuvant is selected from the group consisting of calcium phosphate, aluminum phosphate and aluminum oxyhydroxide.

6. The method according to claim 1, wherein the method of assaying said antigen is the procedure of (a).

7. The method according to claim 6, wherein the primary antibody binds to a region defined by the amino acid sequence of SEQ ID NO: 2, 3 or 4 of the antigen having the SEQ ID NO: 1; or binds to the antigen having the SEQ ID NO: 1.

8. The method according to claim 6, wherein the primary antibody is one or more of murine anti-protective antigen monoclonal antibody clone 2B18, murine anti-protective antigen monoclonal antibody clone RDI-TRK3BA16-C3 (clone C3), murine anti-protective antigen monoclonal antibody clone RDI-TRK3BA16-105 mAb or murine anti-protective antigen monoclonal antibody clone RDI-TRK3BA16-106 mAb.

9. The method according to claim 6, wherein said detectable label is a radiolabel, a fluorescent label, a biotinylated label or an enzyme label.

10. The method according to claim 1, wherein the antigen is detected by the procedure of (b).

11. The method according to claim 10, wherein the primary antibody binds to a region defined by the amino acid sequence of SEQ ID NO: 2, 3 or 4 of the antigen having the SEQ ID NO: 1; or binds to the antigen having the SEQ ID NO: 1.

12. The method according to claim 11, wherein the primary antibody is one or more of murine anti-protective antigen monoclonal antibody clone 2B18, murine anti-protective antigen monoclonal antibody clone RDI-TRK3BA16-C3 (clone C3), murine anti-protective antigen monoclonal antibody clone RDI-TRK3BA16-105 mAb or murine anti-protective antigen monoclonal antibody clone RDI-TRK3BA16-106 mAb.

13. The method according to claim 11, wherein the detectably-labeled probe is an antibody or immunoglobulin binding protein.

14. The method according to claim 13, wherein said detectably-labeled probe is an immunoglobulin binding protein selected from Protein A and Protein G.

15. The method according to claim 13, wherein the detectably-labeled probe is a detectably-labeled secondary antibody for the primary antibody/antigen complex.

16. The method according to claim 15, wherein said secondary antibody is an anti-mouse IgG or an anti-rabbit IgG.

17. The method according to claim 15, wherein the primary antibody binds to a region defined by the amino acid sequence of SEQ ID NO: 2, 3 or 4 of the antigen having the SEQ ID NO: 1; or binds to the antigen having the SEQ ID NO: 1.

18. The method according to claim 17, wherein the primary antibody is one or more of murine anti-protective antigen monoclonal antibody clone 2B18, murine anti-protective antigen monoclonal antibody clone RDI-TRK3BA16-C3 (clone C3), murine anti-protective antigen monoclonal antibody clone RDI-TRK3BA16-105 mAb or murine anti-protective antigen monoclonal antibody clone RDI-TRK3BA16-106 mAb.

## Patentansprüche

1. Verfahren zur Bestimmung der Menge an aktiven Milzbrandschutzantigenen, die an einem pharmakologisch annehmbaren kolloidalen Hilfsstoff vorliegen, umfassend:
a) Kontaktieren einer Suspension aus einem von einem kolloidalen Hilfsstoff adsorbierten aktiven Milzbrandschutzantigen mit einer Lösung oder Suspension aus einem nachweisbar markierten primären Antikörper gegen den altiven Antigen-Antikörper zum Schutz gegen Milzbrand zur Bildung eines aktiven Antigen-Antikörperkomplexes zum Schutz gegen Milzbrand und Messen der nachweisbaren Markierung gegenüber einer Probe aus einem herkömmlichen aktiven pharmazeutischen Produkt; oder
b) Kontaktieren einer Suspension aus einem von einem kolloidalen Hilfsstoff adsorbierten aktiven Milzbrandschutzantigen mit einer Lösung oder Suspension aus einem primären Antikörper gegen das aktive Milzbrandschutzantigen zur Bildung eines aktiven Antigen-Antikörperkomplexes zum Schutz gegen Milzbrand und Kontaktieren des aktiven Antigen-Antikörperkomplexes zum Schutz gegen Milzbrand mit einer nachweisbar markierten Sonde für den aktiven Antigen-Antikörperkomplex gegen Milzbrand und Messen der nachweisbaren Markierung an der Sonde gegenüber einer Probe aus einem herkömmlichen aktiven pharmazeutischen Produkt, wodurch die Menge an aktivem Milzbrandschutzantigen bestimmt wird.

2. Verfahren nach Anspruch 1, wobei das Antigen ein Bestandteil eines Untereinheit-Impfstoffs ist.

3. Verfahren nach Anspruch 1, wobei das Antigen die Aminosäuresequenz der SEQ ID NO: 1 umfasst.

4. Verfahren nach Anspruh 1, wobei der primäre Antikörper ein Antikörper für die Domäne 4 des Milzbrandschutzantigens ist.

5. Verfahren nach Anspruch 1, wobei der kolloidale Hilfsstoff aus Kalziumphosphat, Aluminiumphosphat und Aluminiumoxyhydroxid ausgewählt ist.

6. Verfahren nach Anspruch 1, wobei das Verfahren zur Bestimmung des Antigens das Verfahren nach (a) ist.

7. Verfahren nach Anspruch 6, wobei der primäre Antikörper an eine von der Aminosäuresequenz der SEQ ID NO: 2, 3 oder 4 des Antigens mit der SEQ ID NO: 1 oder an das Antigen mit der SEQ ID NO: 1 bindet.

8. Verfahren nach Anspruch 6, wobei der primäre Antikörper eines oder mehrere der nachfolgenden Gruppe ist: monoklonaler Antikörperklon 2B18 des Maus-Anti-Schutzantigens, monoklonaler Antikörperklon RDI-TRK3BA16-C3 (Klon C3) des Maus-Anti-Schutzantigens, monoklonaler Antikörperklon RDI-TRK3BA16-105 mAb des Maus-Anti-Schutzantigens oder monoklonaler Antikörperklon RDI-TRK3BA16-106 mAb des Maus-Anti-Schutzantigens.

9. Verfahren nach Anspruch 6, wobei die nachweisbare Markierung eine radioaktive Markierung, eine fluoreszierende Markierung, eine biotinylierte Markierung oder eine Enzymmarkierung ist.

10. Verfahren nach Anspruch 1, wobei das Antigen durch das Verfahren nach (b) nachgewiesen wird.

11. Verfahren nach Anspruch 10, wobei der primäre Antikörper an eine von der Aminosäuresequenz der SEQ ID NO: 2, 3 oder 4 des Antigens mit der SEQ ID NO: 1 oder an das Antigen mit der SEQ ID NO: 1 bindet.

12. Verfahren nach Anspruch 11, wobei der primäre Antikörper eines oder mehrere der nachfolgenden Gruppe ist: monoklonaler Antikörperklon 2B18 des Maus-Anti-Schutzantigens, monoklonaler Antikörperklon RDI-TRK3BA16-C3 (Klon C3) des Maus-Anti-Schutzantigens, monoklonaler Antikörperklon RDI-TRK3BA16-105 mAb des Maus-Anti-Schutzantigens oder monoklonaler Antikörperklon RDI-TRK3BA16-106 mAb des Maus-Anti-Schutzantigens.

13. Verfahren nach Anspruch 11, wobei die nachweisbar markierte Sonde ein Antikörper oder Immunglobulin bindendes Protein ist.

14. Verfahren nach Anspruch 13, wobei die nachweisbar markierte Sonde ein Immunglobulin bindendes Protein ist, das aus Protein A und Protein G ausgewählt ist.

15. Verfahren nach Anspruch 13, wobei die nachweisbar markierte Sonde ein nachweisbar markierter sekundärer Antikörper gegen den primären Antikörper/Antigen-Komplex ist.

16. Verfahren nach Anspruch 15, wobei der sekundäre Antikörper ein Anti-Maus-IgG oder Anti-Kaninchen-IgG ist.

17. Verfahren nach Anspruch 15, wobei der primäre Antikörper an eine von der Aminosäuresequenz der SEQ ID NO: 2, 3 oder 4 des Antigens mit der SEQ ID NO: 1 oder an das Antigen mit der SEQ ID NO: 1 bindet.

18. Verfahren nach Anspruch 17, wobei der primäre Antikörper eines oder mehrere der nachfolgenden Gruppe ist: monoklonaler Antikörperklon 2B18 des Maus-Anti-Schutzantigens, monoklonaler Antikörperklon RDI-TRK3BA16-C3 (Klon C3) des Maus-Anti-Schutzantigens, monoklonaler Antikörperklon RDI-TRK3BA16-105 mAb des Maus-Anti-Schutzantigens oder monoklonaler Antikörperklon RDI-TRK3BA16-106 mAb des Maus-Anti-Schutzantigens.

## Revendications

1. Procédé de dosage de la quantité d'antigène protecteur d'anthrax actif présent sur un adjuvant colloïdal pharmacologiquement acceptable, comprenant :
a) la mise en contact d'une suspension d'un antigène protecteur d'anthrax actif adsorbé à un adjuvant colloïdal avec une solution ou suspension d'un anticorps primaire marqué de façon détectable contre l'antigène protecteur d'anthrax actif pour former un complexe antigène protecteur d'anthrax actif-anticorps et la mesure du marqueur détectable par rapport à un échantillon de produit pharmaceutique actif standard ; ou
b) la mise en contact d'une suspension d'un antigène protecteur d'anthrax actif adsorbé à un adjuvant colloïdal avec une solution ou suspension d'un anticorps primaire contre l'antigène protecteur d'anthrax actif pour former un complexe antigène protecteur d'anthrax actif-anticorps et la mise en contact dudit complexe antigène protecteur d'anthrax actif-anticorps avec une sonde marquée de façon détectable pour le complexe antigène protecteur d'anthrax actif-anticorps et la mesure du marqueur détectable sur la sonde par rapport à un échantillon de produit pharmaceutique actif standard, de manière à déterminer la quantité d'antigène protecteur d'anthrax actif

2. Procédé selon la revendication 1, dans lequel l'antigène est un composant d'un vaccin de sous-unité.

3. Procédé de la revendication 1, dans lequel l'antigène comprend la séquence d'acides aminés de SEQ ID NO: 1.

4. Procédé selon la revendication 1, dans lequel l'anticorps primaire est un anticorps pour le domaine 4 de l'antigène protecteur d'anthrax.

5. Procédé selon la revendication 1, dans lequel ledit adjuvant colloïdal est choisi dans le groupe constitué du phosphate de calcium, du phosphate d'aluminium et de l'oxyhydroxyde d'aluminium.

6. Procédé selon la revendication 1, le procédé de dosage dudit antigène étant la procédure de (a).

7. Procédé selon la revendication 6, dans lequel l'anticorps primaire se lie à une région définie par la séquence d'acides aminés de SEQ ID NO: 2, 3 ou 4 de l'antigène ayant la SEQ ID NO: 1 ou se lie à l'antigène ayant la SEQ ID NO: 1.

8. Procédé selon la revendication 6, dans lequel l'anticorps primaire est un ou plusieurs parmi un anticorps monoclonal anti-antigène protecteur murin clone 2B18, un anticorps monoclonal anti-antigène protecteur murin clone RDI-TRK3BA16-C3 (clone C3), un anticorps monoclonal anti-antigène protecteur murin clone RDI-TRK3BA16-105 mAb ou un anticorps monoclonal anti-antigène protecteur murin clone RDI-TRK3BA16-106 mAb.

9. Procédé selon la revendication 6, dans lequel ledit marqueur détectable est un radiomarqueur, un marqueur fluorescent, un marqueur biotinylé ou un marqueur enzymatique.

10. Procédé selon la revendication 1, dans lequel l'antigène est détecté par la procédure de (b).

11. Procédé selon la revendication 10, dans lequel l'anticorps primaire se lie à une région définie par la séquence d'acides aminés de SEQ ID NO: 2, 3 ou 4 de l'antigène ayant la SEQ ID NO: 1 ou se lie à l'antigène ayant la SEQ ID NO: 1.

12. Procédé selon la revendication 11, dans lequel l'anticorps primaire est un ou plusieurs parmi un anticorps monoclonal anti-antigène protecteur murin clone 2B18, un anticorps monoclonal anti-antigène protecteur murin clone RDI-TRK3BA16-C3 (clone C3), un anticorps monoclonal anti-antigène protecteur murin clone RDI-TRK3BA16-105 mAb ou un anticorps monoclonal anti-antigène protecteur murin clone RDI-TRK3BA16-106 mAb.

13. Procédé selon la revendication 11, dans lequel la sonde marquée de façon détectable est un anticorps ou une protéine de liaison d'immunoglobuline.

14. Procédé selon la revendication 13, dans lequel ladite sonde marquée de façon détectable est une protéine de liaison d'immunoglobuline choisie parmi la protéine A et la protéine G.

15. Procédé selon la revendication 13, dans lequel la sonde marquée de façon détectable est un anticorps secondaire marqué de façon détectable pour le complexe anticorps primaire/antigène.

16. Procédé selon la revendication 15, dans lequel ledit anticorps secondaire est un anti-IgG de souris ou un anti-IgG de lapin.

17. Procédé selon la revendication 15, dans lequel l'anticorps primaire se lie à une région définie par la séquence d'acides aminés de SEQ ID NO: 2, 3 ou 4 de l'antigène ayant la SEQ ID NO: 1 ; ou se lie à l'antigène ayant la SEQ ID NO: 1.

18. Procédé selon la revendication 17, dans lequel l'anticorps primaire est un ou plusieurs parmi un anticorps monoclonal anti-antigène protecteur murin clone 2B18, un anticorps monoclonal anti-antigène protecteur murin clone RDI-TRK3BA16-C3 (clone C3), un anticorps monoclonal anti-antigène protecteur murin clone RDI-TRK3BA16-105 mAb ou un anticorps monoclonal anti-antigène protecteur murin clone RDI-TRK3BA16-106 mAb.


### SEQUENCE LISTING

<110> Avecia Biologies Limited
<120> Method for Assaying Antigens
<130> SMC 60740/Wo
<160> 4
<170> PatentIn version 3.3
<210> 1
   <211> 736
   <212> PRT
   <213> Bacillus anthracis
<400> 1
   Met Glu Val Lys Gln Glu Asn Arg Leu Leu Asn Glu Ser Glu Ser Ser
   1               5                   10                  15
   Ser Gln Gly Leu Leu Gly Tyr Tyr Phe Ser Asp Leu Asn Phe Gln Ala
               20                  25                  30
   Pro Met Val Val Thr Ser Ser Thr Thr Gly Asp Leu Ser Ile Pro Ser
           35                  40                  45
   Ser Glu Leu Glu Asn Ile Pro Ser Glu Asn Gln Tyr Phe Gln Ser Ala
       50                  55                  60
   Ile Trp Ser Gly Phe Ile Lys Val Lys Lys Ser Asp Glu Tyr Thr Phe
   65                  70                  75                  80
   Ala Thr Ser Ala Asp Asn His Val Thr Met Trp Val Asp Asp Gln Glu
                   85                  90                  95
   Val Ile Asn Lys Ala Ser Asn Ser Asn Lys Ile Arg Leu Glu Lys Gly
               100                 105                 110
   Arg Leu Tyr Gln Ile Lys Ile Gln Tyr Gln Arg Glu Asn Pro Thr Glu
           115                 120                 125
   Lys Gly Leu Asp Phe Lys Leu Tyr Trp Thr Asp Ser Gln Asn Lys Lys
       130                 135                 140
   Glu Val Ile Ser Ser Asp Asn Leu Gln Leu Pro Glu Leu Lys Gln Lys
   145                 150                 155                 160
   Ser Ser Asn Ser Arg Lys Lys Arg Ser Thr Ser Ala Gly Pro Thr Val
                   165                 170                 175
   Pro Asp Arg Asp Asn Asp Gly Ile Pro Asp Ser Leu Glu Val Glu Gly
               180                 185                 190
   Tyr Thr Val Asp Val Lys Asn Lys Arg Thr Phe Leu Ser Pro Trp Ile
           195                 200                 205
   Ser Asn Ile His Glu Lys Lys Gly Leu Thr Lys Tyr Lys Ser Ser Pro
       210                 215                 220
   Glu Lys Trp Ser Thr Ala Ser Asp Pro Tyr Ser Asp Phe Glu Lys Val
   225                 230                 235                 240
   Thr Gly Arg Ile Asp Lys Asn Val Ser Pro Glu Ala Arg His Pro Leu
                   245                 250                 255
   Val Ala Ala Tyr Pro Ile Val His Val Asp Met Glu Asn Ile Ile Leu
               260                 265                 270
   Ser Lys Asn Glu Asp Gln Ser Thr Gln Asn Thr Asp Ser Glu Thr Arg
           275                 280                 285
   Thr Ile Ser Lys Asn Thr Ser Thr Ser Arg Thr His Thr Ser Glu Val
       290                 295                 300
   His Gly Asn Ala Glu Val His Ala Ser Phe Phe Asp Ile Gly Gly Ser
   305                 310                 315                 320
   Val Ser Ala Gly Phe Ser Asn Ser Asn Ser Ser Thr Val Ala Ile Asp
                   325                 330                 335
   His Ser Leu Ser Leu Ala Gly Glu Arg Thr Trp Ala Glu Thr Met Gly
               340                 345                 350
   Leu Asn Thr Ala Asp Thr Ala Arg Leu Asn Ala Asn Ile Arg Tyr Val
           355                 360                 365
   Asn Thr Gly Thr Ala Pro Ile Tyr Asn Val Leu Pro Thr Thr Ser Leu
       370                 375                 380
   Val Leu Gly Lys Asn Gln Thr Leu Ala Thr Ile Lys Ala Lys Glu Asn
   385                 390                 395                 400
   Gln Leu Ser Gln Ile Leu Ala Pro Asn Asn Tyr Tyr Pro Ser Lys Asn
                   405                 410                 415
   Leu Ala Pro Ile Ala Leu Asn Ala Gln Asp Asp Phe Ser Ser Thr Pro
               420                 425                 430
   Ile Thr Met Asn Tyr Asn Gln Phe Leu Glu Leu Glu Lys Thr Lys Gln
           435                 440                 445
   Leu Arg Leu Asp Thr Asp Gln Val Tyr Gly Asn Ile Ala Thr Tyr Asn
       450                 455                 460
   Phe Glu Asn Gly Arg Val Arg Val Asp Thr Gly Ser Asn Trp Ser Glu
   465 470 475 480
   Val Leu Pro Gln Ile Gln Glu Thr Thr Ala Arg Ile Ile Phe Asn Gly
   485 490 495
   Lys Asp Leu Asn Leu Val Glu Arg Arg Ile Ala Ala Val Asn Pro Ser
   500 505 510
   Asp Pro Leu Glu Thr Thr Lys Pro Asp Met Thr Leu Lys Glu Ala Leu
   515 520 525
   Lys Ile Ala Phe Gly Phe Asn Glu Pro Asn Gly Asn Leu Gln Tyr Gln
   530 535 540
   Gly Lys Asp Ile Thr Glu Phe Asp Phe Asn Phe Asp Gln Gln Thr Ser
   545 550 555 560
   Gln Asn Ile Lys Asn Gln Leu Ala Glu Leu Asn Ala Thr Asn Ile Tyr
   565 570 575
   Thr Val Leu Asp Lys Ile Lys Leu Asn Ala Lys Met Asn Ile Leu Ile
   580 585 590
   Arg Asp Lys Arg Phe His Tyr Asp Arg Asn Asn Ile Ala Val Gly Ala
   595 600 605
   Asp Glu Ser Val Val Lys Glu Ala His Arg Glu Val Ile Asn Ser Ser
   610 615 620
   Thr Glu Gly Leu Leu Leu Asn Ile Asp Lys Asp Ile Arg Lys Ile Leu
   625 630 635 640
   Ser Gly Tyr Ile Val Glu Ile Glu Asp Thr Glu Gly Leu Lys Glu Val
   645 650 655
   Ile Asn Asp Arg Tyr Asp Met Leu Asn Ile Ser Ser Leu Arg Gln Asp
   660 665 670
   Gly Lys Thr Phe Ile Asp Phe Lys Lys Tyr Asn Asp Lys Leu Pro Leu
   675 680 685
   Tyr Ile Ser Asn Pro Asn Tyr Lys Val Asn Val Tyr Ala Val Thr Lys
   690 695 700
   Glu Asn Thr Ile Ile Asn Pro Ser Glu Asn Gly Asp Thr Ser Thr Asn
   705 710 715 720
   Gly Ile Lys Lys Ile Leu Ile Phe Ser Lys Lys Gly Tyr Glu Ile Gly
   725 730 735
<210> 2
   <211> 18
   <212> PRT
   <213> Bacillus anthracis
<400> 2
   Ile Arg Lys Ile Leu Ser Gly Tyr Ile Val Glu Ile Glu Asp Thr Glu
   1               5                   10                  15
   Gly Leu
<210> 3
   <211> 18
   <212> PRT
   <213> Bacillus anthracis
<400> 3
   Arg Tyr Asp Met Leu Asn Ile Ser Ser Leu Arg Gln Asp Gly Lys Thr
   1               5                   10                  15
   Phe Ile
<210> 4
   <211> 15
   <212> PRT
   <213> Bacillus anthracis
<400> 4
   Phe Ile Asp Phe Lys Lys Tyr Asn Asp Lys Leu Pro Leu Tyr Ile
   1               5                   10                  15